# EUROPEAN PATENT SPECIFICATION

(11) **EP 1 596 881 B1**
(45) Date of publication and mention of the grant of the patent: **10.12.2008**
(21) Application number: 03708393.8
(22) Date of filing: 07.02.2003
(51) Int. Cl.: A61K 38/24, A61P 15/02, A61P 15/08

(54) **USE OF HUMAN CHORIONIC GONADOTROPIN IN THE TREATMENT OF SYMPTOMS CAUSED BY ENDOMETRIOSIS**
VERWENDUNG DES MENSCHLICHEN CHORIONGONADOTROPINS IN DER BEHANDLUNG VON ENDOMETRIOSE-SYMPTOMEN
UTILISATION DE LA GONADOTROPHINE CHORIONIQUE HUMAINE DANS LE TRAITEMENT DES SYMPTOMES CAUSES PAR L'ENDOMETRIOSE

(43) Date of publication of application: 23.11.2005
(73) Proprietor: Austria Wirtschaftsservice Gesellschaft mit beschränkter Haftung, 1030 Vienna (AT); Huber, Ambros Valentin, 1080 Vienna (AT)
(72) Inventor: HUBER, Ambros, Valentin, A-1080 Vienne (AT)
(74) Representative: Sonn & Partner Patentanwälte
(86) International application number: PCT/IB2003/001066
(87) International publication number: WO 2004/069271

(56) References cited:
- EP-A- 0 136 781
- EP-A- 1 022 027
- WO-A-00/67778
- WO-A-03/022302
- US-A- 4 196 123

## Description

### Field of the invention.

This invention relates to a second medical use of human chorionic gonadotropin in a new method for the therapeutic management of chronic and acute pelvic pain, pre-menstrual syndrom (PMS), dysmenorrhea and dyspareunia caused by endometriosis.
It was found by us that hCG, commonly used for the induction of ovulations and for the treatment of luteal insufficiency, also reduces the symptoms caused by endometriosis.

### Background of the invention and prior art.

Human chorionic gonadotropin (hCG) is a glycoprotein (molecular weight of about 36.700), that is composed of an α- and a β-subunit. It is secreted by the syncytiophoblast. The rate of secretion of hCG increases rapidly in the first few weeks of pregnancy, and maximal levels are attained in maternal blood and urine at approximately 10 weeks ad gestation.
The physiological role of hCG in human pregnancy is not fully defined. hCG acts as a luteotropin to maintain the corpus luteum and serves to convert the corpus luteum of menstruation to the corpus luteum of pregnancy through its capacity to stimulate the secretion of progesterone and relaxin.

It was found that hCG inhibits the growth of benign, borderline and malignant ovarian epithelial cell lines (Tourgeman et. al., 2002).

hCG and the hCG - subunit also inhibit the proliferation of cell lines derived from Kaposi's sarcoma (KS). Regression of Kaposi's sarcoma has been shown in two women during pregnancy, where the level of this hormone is high (Lunardi-lskandar et al. , 1995) .

Albini and Orengo (1996) found that KS cells membranes above defined contain binding sites of β-core and deglycosylated hCG and it was concluded that β-core itself and/or deglycosylated hCG is the agent responsible for the in vivo and in vitro biological activities found by Lunardi-lskandar (1995).

Endometriosis is defined by the presence and proliferation of endometric tissue (glands and stromal) outside the endometrial cavity as well as in the myometrium. Acute or chronic pelvic pain, dysmenorrhea, dyspareunia, pre-menstrual syndrom and infertility perform the most frequent clinical symptoms. The ectopic endometric tissue responds to ovarian hormones undergoing cyclic changes. The cyclical bleeding from the endometric deposit contributes to a local inflammatory reaction. Endometriosis commonly affects women during their childbearing years.

Treatments of endometriosis include operative laparoscopy with resection or ablation of endometric implant, and temporary gonadal suppression by danazol, progesta-gens, continuous oral contraceptive tablets, or luteinizing hormone-releasing hormone (LHRH) agonists.

WO 00/67778 A relates to the use of gonadotropins for the treatment of infertility. EP 1 022 027 A discloses TNF antagonists and their use in endometriosis. In US 4,196,123 A hybrid chorionic gonadotropin and methods for stimulating ovulation using the same are described.

### Summary of the invention.

It was observed that in patients with endometriosis, by whom the endometric implants were removed by operative laparoscopy and who, due to persistent pain, consecutively were "down-regulated" with LHRH agonists and submitted to a temporary gonadal suppression by danazol and progestagens, the pain remained. I found that a treatment with hCG (Pregnyl®) injections resulted in an immediate and remaining improvement of the situation and in the following in a full disappearance of the pain.

Accordingly, the present invention relates to the use of hCG for the preparation of a medicament for the treatment of chronic pelvic pain, PMS, dysmenorrhea and dyspareunia caused by endometriosis.

### Detailed description of the invention.

Up until now, all allowed and commonly applied treatments of symptoms caused by endometriosis have no satisfying result.
Clinical experience showed that only a pregnancy can result in a permanent healing of these symptoms.

Based on these clinical experience, patients which have been submitted to all possible medical treatments of the symptoms caused by endometriosis and still were in pain, have been treated with hCG.

The hCG is administrated over a period of 3 days to 60 months, with a concentration of 1 I.E. to 500.000 I.E., every day, every 2^{nd}, 3^{rd}, 4^{th}, 5^{th}, 6^{th}, or 7^{th} day.

The hCG can be injected intra-muscular or can be administrated orally.

According to the invention a hCG is administrated for 2 weeks every 3^{rd} day with a concentration of 750 I.E., consequetively for 2 weeks every 3^{rd} day with a concentration of 1.500 I.E., followed by 2 weeks every 4^{th} day with a concentration of 3.000 I.E., the following 2 weeks every 5^{th} day with a concentration of 5.000 I.E., the following 2 weeks every 4^{th} day with a concentration of 3.000 I.E., followed by 2 weeks every 3^{rd} day with a concentration of 1.500 I.E., and the last 2 weeks every 3^{rd} day with a concentration of 750 I.E. (Diagram1).

According to another aspect of the invention, the hCG is administrated at day 1 with a concentration of 750 I.E., at day 3 with a concentration of 1.000 I.E., at day 5 with a concentration of 1.500 I.E., at day 8 with a concentration of 1.750 I.E., at day 12 with a concentration of 2.000 I.E., at day 17 with a concentration of 5.000 I.E., at day 24 with a concentration of 7.500 I.E., at day 31 with a concentration of 10.000 I.E., at day 38 with a concentration of 5.000 I.E., at day 48 with a concentration of 2.500 I.E., at day 55 with a concentration of 2.000 I.E., the following 4 weeks with a concentration of 1.500 I.E. once a week, followed by 4 weeks with a concentration of 1.000 I.E. once a week and finally followed by 4 weeks with a concentration of 750 I.E. once a week (Diagram 2).

According to another aspect of the invention, the hCG is administrated for 2 weeks every 3^{rd} day with a concentration of 750 I.E., with a concentration of 1.500 I.E. every 3^{rd} day in the following 2 weeks, followed by an administration with a concentration of 3.000 I.E. every 3^{rd} day for 2 weeks, followed by an administration with a concentration of 5.000 I.E. every 3^{rd} day for the following 2 weeks, during the following 2 weeks with a concentration of 3.000 I. E. every 3^{rd} day, with a concentration of 1.500 I.E. every 3^{rd} day for the following 12 weeks, followed by administration with a concentration of 750 I.E. 2 times a week for a period of 30 weeks (Diagram 3).

According to another aspect of the invention, the hCG is administrated for a period of 4 weeks with a concentration of 750 I.E. - 5.000 I.E. every 2^{nd}, 3^{rd}, or 4^{th} day, followed by a period of 4 weeks with an administration every 2^{nd}, 3^{rd} or 4^{th} day of a concentration of 1.500 I.E. - 10.000 I.E., followed by an administration of a concentration of 1.000 I.E. - 5.000 I.E. every 3^{rd} of 4^{th} day over a period of 10 months. Preferably, the hCG is administrated for a period of 4 weeks with a concentration of 750 I.E. - 2.500 I.E. every 3^{rd} or 4^{th} day, followed by a period of 4 weeks with an administration of 1.500 I.E. - 5.000 I.E. every 3^{rd} or 4th day, followed by an administration of 1.000 I.E. - 2.500 I.E. every 3^{rd} or 4^{th} day over a period of 10 months.
More preferably, the hCG is administrated for a period of 4 weeks with a concentration of 750 I.E. every 4^{th} day, followed by a period of 4 weeks with an administration of 1.500 I.E. every 4^{th} day, followed by an administration of 1.000 I.E. every 4^{th} day over a period of 10 months.

According to another aspect of the invention, in analogy to the situation during pregnancy, the hCG is administrated over a period of 9 months whereby the hCG is administrated once or twice a week in week 1 to 4 week with a concentration of 100 I.E. - 2.000 I.E., in week 5-6 with a concentration of 2.500 I.E. - 3.500 I.E., in week 7-8 with a concentration of 4.000 I.E. - 20.000 I.E., in week 9-10 with a concentration of 30.000 I.E. - 100.000 I.E., in week 11-14 with a concentration of 5.000 I.E - 30.000 I.E., in week 15-20 with a concentration of 1.000 I.E. - 3.000 I.E., in week 21-40 with a concentration of 100 I.E. - 500 I.E. (Diagram 4).

According to another aspect of the invention, the hCG is administrated continuously as long as the patient suffers from pain, whereby the therapy is restricted to a period of 4 weeks to 5 years, every day with a concentration of 1 I.E. - 500 I.E., whereby in case of acute pain the concentration of the hCG administrated is augmented to 5.000 - 500.000 I.E. for a period of 1, 2, 3, 4, or 5 days, period in which the hCG is administrated every day or every 2^{nd} day.

According to another aspect of the invention, for the treatment of PMS and dysmenorrhea the hCG is administrated 3 days before menstruation and/or during menstruation with a concentration of 750 I.E. - 5.000 I.E. every day. Preferably a concentration of 1.500 I.E. is administrated every day.

### Literature:

Albini A, Paglieri I, Orengo G, Carlone S, Aluigi MG, DeMarchi R, Matteucci C, Mantovani A, Carozzi F, Donini S, Benelli R, 1997, "The beta-core fragment of human chorionic gonadotrophin inhibits growth of Kaposi's sarcoma-derived cells and a new immortalized Kaposi's sarcoma cell line.", AIDS 1997 May;11(6):713-21.

Tourgeman DE, Lu JJ, Boostanfar R, Amezcua C, Felix JC, Paulson RJ, "Human chorionic gonadotropin suppresses ovarian epithelial neoplastic cell proliferation in vitro.",Fertil Steril 2002 Nov;78(5):1096-9.

Lunardi-Iskandar Y, Bryant JL, Zeman RA, Lam VH, Samaniego F, Besnier JM, Hermans P, Thierry AR, Gill P, Gallo RC, "Tumorigenesis and metastasis of neoplastic Kaposi's sarcoma cell line in immunodeficient mice blocked by a human pregnancy hormone." Nature 1995 May 4;375(6526):64-8.

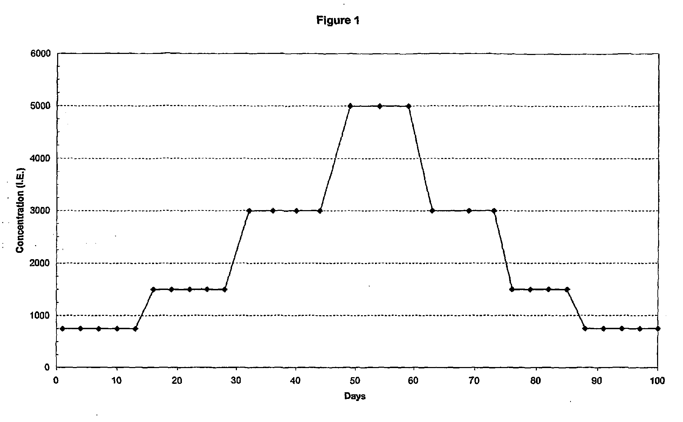

Fig.1.: Change of the concentration of the medicament administrated with time.

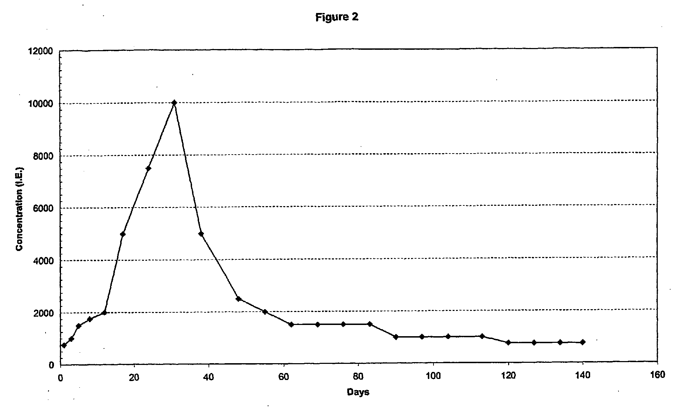

Fig.2.: Change of the concentration of the medicament administrated with time.

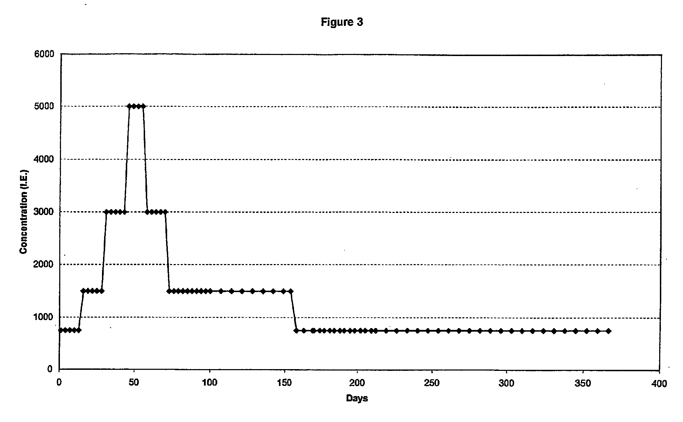

Fig.3.: Change of the concentration of the medicament administrated with time.

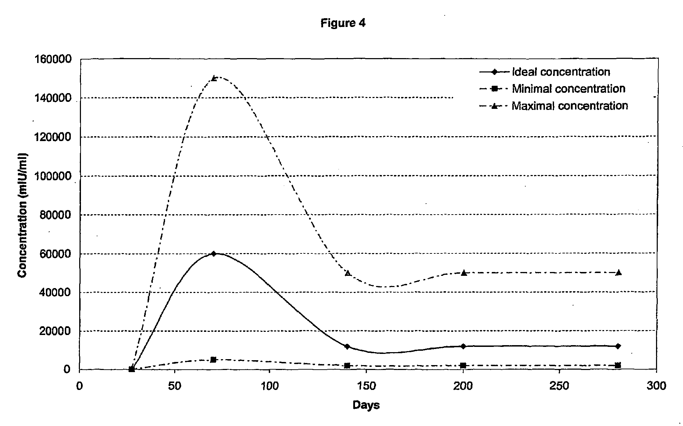

Fig.4.: Change of the ideal, maximal and minimal concentration of the medicament administrated with time

## Claims

1. : Use of human chorionic gonadotropin (hcg) for the preparation of a medicament for the treatment of chronic pelvic pain, pre-menstrual syndrome (PMS), dysmenorrhea and dyspareunia caused by endometriosis.

2. : Use according to claim 1, wherein the medicament can be supplied by intramuscular injection or by oral administration, over a period of 3 days to 60 months, with a concentration of 1 I.E. to 500.000 I.E., every day, every 2nd, 3rd, 4th, 5th, 6th, or 7th day.

3. : Use according to claim 1 or 2, wherein the medicament is administrated for 2 weeks every 3rd day with a concentration of 750 I.E., consequetively for 2 weeks every 3rd day with a concentration of 1.500 I.E., followed by 2 weeks every 4th day with a concentration of 3.000 I.E., the following 2 weeks every 5th day with a concentration of 5.000 I.E., the following 2 weeks every 4th day with a concentration of 3.000 I.E., followed by 2 weeks every 3rd day with a concentration of 1.500 I.E., and the last 2 weeks every 3rd day with a concentration of 750 I.E.

4. : Use according to claim 1 or 2, wherein the medicament is administrated at day 1 with a concentration of 750 I.E., at day 3 with a concentration of 1.000 I.E., at day 5 with a concentration of 1.500 I.E., at day 8 with a concentration of 1.750 I.E., at day 12 with a concentration of 2.000 I.E., at day 17 with a concentration of 5.000 I.E., at day 24 with a concentration of 7.500 I.E., at day 31 with a concentration of 10.000 I.E., at day 38 with a concentration of 5.000 I.E., at day 48 with a concentration of 2.500 I.E., at day 55 with a concentration of 2.000 I.E., the following 4 weeks with a concentration of 1.500 I.E. once a week, followed by 4 weeks with a concentration of 1.000 I.E. once a week and finally followed by 4 weeks with a concentration of 750 I.E. once a week.

5. : Use according to claim 1 or 2, wherein the medicament is administrated for 2 weeks every 3rd day with a concentration of 750 I.E., with a concentration of 1.500 I.E. every 3rd day in the following 2 weeks, followed by an administration with a concentration of 3.000 I.E. every 3rd day for 2 weeks, followed by an administration with a concentration of 5.000 I.E. every 3rd day for the following 2 weeks, during the following 2 weeks with a concentration of 3.000 I. E. every 3rd day, with a concentration of 1.500 I.E. every 3rd day for the following 12 weeks, followed by administration with a concentration of 750 I.E. 2 times a week for a period of 30 weeks.

6. : Use according to claim 1 or 2, wherein the medicament is administrated for a period of 4 weeks at a concentration of 750 I.E. - 5.000 I.E. every 2nd, 3rd, or 4th day, followed by a period of 4 weeks with an administration every 2nd, 3rd or 4th day of 1.500 I.E. - 10.000 I.E., followed by an administration of 1.000 I.E. - 5.000 I.E. every 3rd of 4th day over a period of 10 months.

7. : Use according to claim 6, wherein the medicament is administrated for a period of 4 weeks at a concentration of 750 I.E. - 2.500 I.E. every 3rd or 4th day, followed by a period of 4 weeks with an administration of 1.500 I.E. - 5.000 I.E. every 3rd or 4th day, followed by an administration of 1.000 I.E. - 2.500 I.E. every 3rd or 4th day over a period of 10 months.

8. : Use according to claim 7, wherein the medicament is administrated for a period of 4 weeks at a concentration of 750 I.E. every 4th day, followed by a period of 4 weeks with an administration of 1.500 I.E. every 4th day, followed by an administration of 1.000 I.E. every 4th day over a period of 10 months.

9. : The use according to claim 1 or 2, wherein the medicament is administrated over a period of 9 months, whereby it is administrated once or twice a week in week 1 to 4 week with a concentration of 100 I.E. - 2.000 I.E., in week 5-6 with a concentration of 2.500 I.E. - 3.500 I.E., in week 7-8 with a concentration of 4.000 I.E. - 20.000 I.E., in week 9-10 with a concentration of 30.000 I.E. - 100.000 I.E., in week 11-14 with a concentration of 3.000 I.E. - 30.000 I.E., in week 15-20 with a concentration of 1.000 I.E. - 3.000 I.E., in week 21-40 with a concentration of 100 I.E. - 500 I.E.

10. : The use according to claim 1 or 2, whereby the medicament is administrated continuously as long as the patient suffers from pain, whereby the therapy is restricted to a period of 4 weeks to 5 years, every day with a concentration of 1 I.E. - 500 I.E., whereby in case of acute pain the concentration of the medicament administrated is augmented to 5.000 - 500.000 I.E. for a period of 1, 2, 3, 4, or 5 days, period in which the medicament is administrated every day or every 2nd day.

11. : The use according to claim 1 or 2, wherein said symptom is PMS or dysmenorrhea, and the medicament is administrated 3 days before menstruation at a concentration of 750 I.E. - 5.000 I.E. every day.

12. : The use according to claim 1 or 2, wherein said symptom is PMS or dysmenorrhea, and the medicament is administrated 3 days before menstruation and during menstruation at a concentration of 750 I.E. - 5.000 I.E. every day.

13. : Use according to claim 11 or 12, wherein the medicament is administrated every day at a concentration of 1.500 I.E.

14. : The use according to any one of claims 1 to 13, wherein the medicament is administrated by intra-muscular injection.

15. : The use according to any one of claims 1 to 13, wherein the medicament is administrated orally.

## Patentansprüche

1. Verwendung von humanem Choriongonadotropin (hcg) zur Herstellung eines Medikaments zur Behandlung von chronischen Beckenschmerzen, von prämenstruellem Syndrom (PMS), Dysmenorrhoe und Dyspareunie, ausgelöst durch Endometrose.

2. Verwendung nach Anspruch 1, wobei das Medikament durch intramuskuläre Injektion oder durch orale Verabreichung über einen Zeitraum von 3 Tagen bis 60 Monate in einer Konzentration von 1 I.E. bis 500.000 I.E. täglich oder jeden zweiten, dritten, vierten, fünften, sechsten oder siebten Tag zugeführt, wird.

3. Verwendung nach Anspruch 1 oder 2, wobei das Medikament 2 Wochen lang jeden dritten Tag in einer Konzentration von 750 I.E, danach 2 Wochen lang jeden dritten Tag in einer Konzentration von 1.500 I.E., danach 2 Wochen lang jeden vierten Tag in einer Konzentration von 3.000 I.E., die folgenden zwei Wochen jeden fünften Tag in einer Konzentration von 5.000 I.E., die folgenden 2 Wochen jeden vierten Tag in einer Konzentration von 3.000 I.E., danach 2 Wochen lang jeden dritten Tag in einer Konzentration von 1.500 I.E. und die letzten zwei Wochen jeden dritten Tag in einer Konzentration von 750 I.E. verabreicht wird.

4. Verwendung nach Anspruch 1 oder 2, wobei das Medikament an Tag 1 in einer Konzentration von 750 I.E., an Tag 3 in einer Konzentration von 1.000 I.E., an Tag 5 in einer Konzentration von 1.500 I.E., an Tag 8 in einer Konzentration von 1.750 I.E., an Tag 12 in einer Konzentration von 2.000 I.E., an Tag 17 in einer Konzentration von 5.000 I.E., an Tag 24 in einer Konzentration von 7.500 I.E., an Tag 31 in einer Konzentration von 10.000 I.E., an Tag 38 in einer Konzentration von 5.000 I.E., an Tag 48 in einer Konzentration von 2.500 I.E., an Tag 55 in einer Konzentration von 2.000 I.E., gefolgt von vier Wochen wöchentlich in einer Konzentration von 1.500 I.E., danach 4 Wochen lang wöchentlich in einer Konzentration von 1.000 I.E. und schließlich gefolgt von vier Wochen wöchentlich in einer Konzentration von 750 I.E. verabreicht wird.

5. Verwendung nach Anspruch 1 oder 2, wobei das Medikament zwei Wochen lang jeden dritten Tag in einer Konzentration von 750 I.E., in den folgenden Wochen jeden dritten Tag in einer Konzentration von 1.500 I.E, danach zwei Wochen lang jeden dritten Tag in einer Konzentration von 3.000 I.E., danach zwei Wochen lang jeden dritten Tag in einer Konzentration von 5.000 I.E., während der folgenden zwei Wochen jeden dritten Tag in einer Konzentration von 3.000 I.E., die folgenden 12 Wochen lang jeden dritten Tag in einer Konzentration von 1.500 I.E. und danach 30 Tage lang zweimal wöchentlich in einer Konzentration von 750 I.E. verabreicht wird.

6. Verwendung nach Anspruch 1 oder 2, wobei das Medikament über einen Zeitraum von vier Wochen jeden zweiten, dritten oder vierten Tag in einer Konzentration von 750 I.E. bis 5.000 I.E., danach über einen Zeitraum von vier Wochen jeden zweiten, dritten oder vierten Tag in einer Konzentration von 1.500 I.E. bis 10.000 I.E. und danach über einen Zeitraum von zehn Monaten jeden dritten oder vierten Tag in einer Konzentration von 1.000 bis 5.000 I.E. verabreicht wird.

7. Verwendung nach Anspruch 6, wobei das Medikament über einen Zeitraum von vier Wochen jeden dritten oder vierten Tag in einer Konzentration von 750 I.E. bis 2.500 I.E., danach über einen Zeitraum von vier Wochen jeden dritten oder vierten Tag in einer Konzentration von 1.500 I.E. bis 5.000 I.E., danach über einen Zeitraum von zehn Monaten jeden dritten oder vierten Tag in einer Konzentration von 1.000 I.E. bis 2.500 I.E. verabreicht wird.

8. Verwendung nach Anspruch 7, wobei das Medikament über einen Zeitraum von vier Wochen jeden vierten Tag in einer Konzentration von 750 I.E., danach über einen Zeitraum von vier Wochen jeden vierten Tag in einer Konzentration von 1.500 I.E. und danach über einen Zeitraum von zehn Monaten jeden vierten Tag in einer Konzentration von 1.000 I.E. verabreicht wird.

9. Verwendung nach Anspruch 1 oder 2, wobei das Medikament über einen Zeitraum von neun Monaten verabreicht wird, wobei es einmal oder zweimal wöchentlich in Woche 1 bis 4 in einer Konzentration von 100 I.E. bis 2.000 I.E., in Woche 5 bis 6 in einer Konzentration von 2.500 I.E. bis 3.500 I.E., in Woche 7 bis 8 in einer Konzentration von 4.000 I.E. bis 20.000 I.E., in Woche 9 bis 10 in einer Konzentration von 30.000 I.E. bis 100.000 I.E., in Woche 11 bis 14 in einer Konzentration von 3.000 I.E. bis 30.000 I.E., in Woche 15 bis 20 in einer Konzentration von 1.000 I.E. bis 3.000 I.E. und in Woche 21 bis 40 in einer Konzentration von 100 I.E. bis 500 I.E. verabreicht wird.

10. Verwendung nach Anspruch 1 oder 2, wobei das Medikament so lange kontinuierlich verabreicht wird, solange der Patient Schmerzen hat, wobei die Therapie auf einen Zeitraum von vier Wochen bis 5 Jahre mit einer täglichen Konzentration von 1 I.E. bis 500 I.E. beschränkt ist, wobei im Falle von akuten Schmerzen die Konzentration des verabreichten Medikaments über einen Zeitraum von einem Tag oder zwei, drei, vier oder fünf Tagen auf 5.000 I.E. bis 500.000 I.E. erhöht wird, ein Zeitraum, in dem das Medikament jeden oder jeden zweiten Tag verabreicht wird.

11. Verwendung nach Anspruch 1 oder 2, wobei das Symptom PMS oder Dysmenorrhoe ist, und wobei das Medikament drei Tage vor der Menstruation täglich in einer Konzentration von 750 I.E. bis 5.000 I.E. verabreicht wird.

12. Verwendung nach Anspruch 1 oder 2, wobei das Symptom PMS oder Dysmenorrhoe ist, und wobei das Medikament drei Tage vor der Menstruation und während der Menstruation täglich in einer Konzentration von 750 I.E. bis 5.000 I.E. verabreicht wird.

13. Verwendung nach Anspruch 11 oder 12, wobei das Medikament täglich in einer Konzentration von 1.500 I.E. verabreicht wird.

14. Verwendung nach einem der Ansprüche 1 bis 13, wobei das Medikament durch intramuskuläre Injektion verabreicht wird.

15. Verwendung nach einem der Ansprüche 1 bis 13, wobei das Medikament oral verabreicht wird.

## Revendications

1. Utilisation de gonadotrophine chorionique humaine (gch) pour la préparation d'un médicament pour le traitement des douleurs pelviennes chroniques, du syndrome prémenstruel (SPM), de la dysménorrhée et de la dysparéunie causées par l'endométriose.

2. Utilisation selon la revendication 1, dans laquelle le médicament peut être administré par injection intramusculaire ou par administration orale, sur une période de 3 jours à 60 mois, à une concentration allant de 1 à 500 000 LE., chaque jour, tous les 2 jours, 3 jours, 4 jours, 5 jours, 6 jours ou 7 jours.

3. Utilisation selon la revendication 1 ou 2, dans laquelle le médicament est administré pendant 2 semaines tous les 3 jours à une concentration de 750 I.E., puis pendant 2 semaines tous les 3 jours à une concentration de 1 500 LE., suivies par 2 semaines tous les 4 jours à une concentration de 3 000 I.E., les 2 semaines suivantes tous les 5 jours à une concentration de 5 000 LE., les 2 semaines suivantes tous les 4 jours à une concentration de 3 000 LE., suivi de 2 semaines tous les 3 jours à une concentration de 1 500 I.E., et les 2 dernières semaines tous les 3 jours à une concentration de 750 I.E..

4. Utilisation selon la revendication 1 ou 2, dans laquelle le médicament est administré au jour 1 à une concentration de 750 I.E., au jour 3 à une concentration de 1 000 I.E., au jour 5 à une concentration de 1 500 LE., au jour 8 à une concentration de 1 750 LE., au jour 12 à une concentration de 2 000 I.E., au jour 17 à une concentration de 5 000 LE., au jour 24 à une concentration de 7 500 I.E., au jour 31 à une concentration de 10 000 I.E., au jour 38 à une concentration de 5 000 LE., au jour 48 à une concentration de 2 500 LE., au jour 55 à une concentration de 2 000 LE., les 4 semaines suivantes à une concentration de 1 500 I.E. une fois par semaine, suivies de 4 semaines à une concentration de 1 000 I.E. une fois par semaine et suivies enfin de 4 semaines à une concentration de 750 I.E. une fois par semaine.

5. Utilisation selon la revendication 1 ou 2, dans laquelle le médicament est administré pendant 2 semaines tous les 3 jours à une concentration de 750 I.E., à une concentration de 1 500 LE. tous les 3 jours pendant les 2 semaines suivantes, suivies d'une administration à une concentration de 3 000 I.E. tous les 3 jours pendant 2 semaines, suivies d'une administration à une concentration de 5 000 I.E. tous les 3 jours pendant les 2 semaines suivantes, pendant les deux semaines suivantes à une concentration de 3 000 I.E. tous les 3 jours, à une concentration de 1 500 I.E. tous les 3 jours pendant les 12 semaines suivantes, suivies d'une administration à une concentration de 750 I.E. 2 fois par semaine pendant une période de 30 semaines.

6. Utilisation selon la revendication 1 ou 2, dans laquelle le médicament est administré pendant une période de 4 semaines à une concentration de 750 I.E. à 5 000 I.E. tous les 2, 3 ou 4 jours, suivis d'une période de 4 semaines avec une administration tous les 2, 3 ou 4 jours de 1 500 I.E. à 10 000 I.E., suivie d'une administration de 1 000 I.E. à 5 000 I.E. tous les 3 ou 4 jours sur une période de 10 mois.

7. Utilisation selon la revendication 6, dans laquelle le médicament est administré pendant une période de 4 semaines à une concentration de 750 I.E. à 2 500 I.E. tous les 3 ou 4 jours, suivie d'une période de 4 semaines avec une administration de 1 500 I.E. à 5 000 I.E. tous les 3 ou 4 jours, suivie d'une administration de 1 000 I.E. à 2 500 I.E. tous les 3 ou 4 jours sur une période de 10 mois.

8. Utilisation selon la revendication 7, dans la quelle le médicament est administré pendant une période de 4 semaines à une concentration de 750 I.E. tous les 4 jours, suivi d'une période de 4 semaines avec une administration de 1 500 I.E. tous les 4 jours, suivi d'une administration de 1 000 I.E. tous les 4 jours sur une période de 10 mois.

9. Utilisation selon la revendication 1 ou 2, dans laquelle le médicament est administré pendant une période de 9 mois, étant administré 1 à 2 fois par semaine dans les semaines 1 à 4 à une concentration de 100 I.E. à 2 000 I.E., dans les semaines 5 à 6 à une concentration de 2 500 I.E. à 3 500 I.E., dans les semaines 7 à 8 à une concentration de 4 000 I.E. à 20 000 I.E., dans les semaines 9 à 10 à une concentration de 30 000 I.E. à 100 000 I.E., dans les semaines 11 à 14 à une concentration de 3 000 I.E. à 30 000 I.E., dans les semaines 15 à 20 à une concentration de 1 000 I.E. à 3 000 I.E., et dans les semaines 21 à 40 de 100 I.E. à 500 I.E..

10. Utilisation selon la revendication 1 ou 2, dans laquelle le médicament est administré en continu tant que le patient souffre de douleurs, le traitement étant restreint à une période de 4 semaines à 5 ans, tous les jours à une concentration de 1 I.E. à 500 LE., la concentration du médicament administré étant augmentée en cas de douleurs jusqu'à 5 000 I.E. à 500 000 I.E. pendant une période de 1, 2, 3, 4, ou 5 jours, période durant laquelle le médicament est administré chaque jour ou tous les 2 jours.

11. Utilisation selon la revendication 1 ou 2, dans laquelle ledit symptôme est le SPM ou la dysménorrhée, le médicament étant administré 3 jours avant la menstruation à une concentration de 750 I.E. à 5 000 I.E. chaque jour.

12. Utilisation selon la revendication 1 ou 2, dans laquelle ledit symptôme est le SPM ou la dysménorrhée, le médicament étant administré 3 jours avant la menstruation et pendant la menstruation à une concentration de 750 I.E. à 5 000 I.E. chaque jour.

13. Utilisation selon la revendication 11 ou 12, dans laquelle le médicament est administré chaque jour à une concentration de 1 500 I.E.

14. Utilisation selon l'une quelconque des revendications 1 à 13, dans laquelle le médicament est administré par injection intramusculaire.

15. Utilisation selon l'une quelconque des revendications 1 à 13, dans laquelle le médicament est administré par voie orale.
